# EUROPEAN PATENT APPLICATION

(11) **EP 3 653 144 A1**
(43) Date of publication of application: **20.05.2020**
(21) Application number: 19207339.3
(22) Date of filing: 06.11.2019
(51) Int. Cl.: A61B 17/29, A61B 17/3203, A61B 18/14

(54) **DISSECTION DEVICE FOR TISSUE**

(30) Priority: 15.11.2018 KR 20180140644
(71) Applicant: Kim, Wook Hwan, Suwon-si 16517 (KR); Meditech Inframed Co., Ltd., Seoul 05413 (KR)
(72) Inventor: KIM, Wook Hwan, 16517 Suwon-si (KR)
(74) Representative: Zardi, Marco

(57) **Abstract**

The present invention relates to a tissue dissection device used during surgery, and more particularly, to a dissection device for tissue to apply a dissecting pressure to tissue layers having an anatomical plane, so as to easily and accurately find the anatomical plane of tissue to be dissected, and safely, easily and quickly dissect human tissue without a risk of tissue damage.

## Description

### BACKGROUND OF THE INVENTION

### 1. Field of the Invention

The present invention relates to a dissection device for tissue to easily and quickly dissect a human tissue during surgery.

### 2. Description of the Related Art

A human tissue is composed of several layers of tissues. Anatomically, a boundary portion between the layered tissues is defined as an anatomical plane.

The boundary portion of the tissue forming the anatomical plane is composed of fibrous proteins such as collagen, elastin, and reticulin, and holds the layered tissues relatively weakly. Accordingly, the tissues may be easily torn and separated.

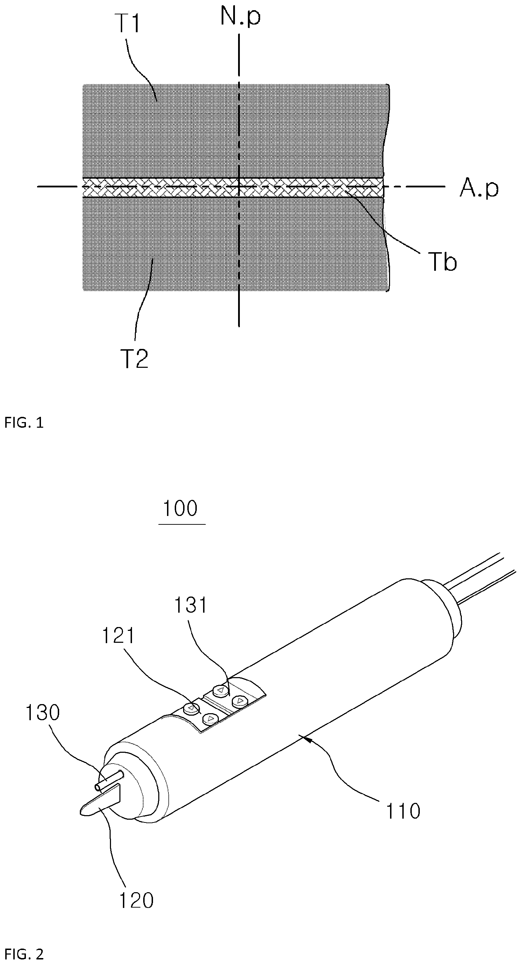

FIG. 1 briefly illustrates a tissue structure in which a tissue T1, a tissue T2, and a boundary tissue Tb are layered. A resection, which is required during surgery, of a tissue at a surgical site may divided into a resection of non-anatomical plane (N.p) and a resection of anatomical plane (A.p).

The resection of non-anatomical plane (N.p) denotes to incise a tissue across the anatomical plane (A.p), for example, to secure a surgical window or the like. The resection of anatomical plane (A.p) denotes to dissect a tissue along the anatomical plane (A.p) to expose or separate a tissue to be dissected. Unlike the resection of non-anatomical plane (N.p), the resection of anatomical plane (A.p) is to separate the tissues on both sides of the boundary portion by tearing or removing the boundary tissue (Tb) that is relatively soft and loose, so that a risk of damaging to tissues or bleeding is minimized.

Accordingly, it is most desirable to accurately find out the anatomical plane (A.p) and quickly dissect the tissue along the anatomical pane (A.p) so as to implement the shortening of operation time, the safety of surgery, or the like.

### SUMMARY OF THE INVENTION

Embodiments of the present invention provide a dissection device for tissue to easily and accurately find out an anatomical plane of a tissue to be dissected during surgery and easily and quickly dissect the tissue along the anatomical plane.

To this end, there is disclosed a tissue dissection device including: a device body unit to be gripped and manipulated; a cauterization electrode provided at a front end of the device body unit to receive electric power and perform electro-cauterization; and a dissecting pressure injection tube provided in the device body unit to apply a dissecting pressure to a front of the cauterization electrode to open the tissue.

In addition, there is disclosed a tissue dissection device which includes: a device body unit to be gripped and manipulated; a dissecting operation performing unit including a support tube extending forward from the device body unit, and a forceps head provided at a front end of the support tube to perform a dissecting operation on the tissue; and a dissecting pressure applying unit including a dissecting pressure injection tube extending forward from the device body unit so as to apply a dissecting pressure to open the tissue.

According to the present invention, a dissecting pressure is applied to tissue layers having an anatomical plane, so that the anatomical plane of a tissue to be dissected can be easily and accurately found, and a human tissue can be safely, easily and quickly dissected without a risk of tissue damage. Accordingly, the operation time can be shortened, and the safety of surgery can be improved.

### BRIEF DESCRIPTION OF THE DRAWINGS

FIG. 1 is a view schematically illustrating a human tissue.
FIGS. 2 and 3 are views illustrating a blade type tissue dissection device and a probe type tissue dissection device according to a first embodiment.
FIGS. 4 and 5 are views schematically illustrating dissecting states of an anatomical plane using the tissue dissection device according to the first embodiment.
FIGS. 6 and 7 are views illustrating configurations and position adjustments in which a cauterization electrode is formed outside or inside the dissecting pressure injection tube.
FIG. 8 is a view illustrating a configuration in which the dissecting pressure injection tube is dually formed inside and outside the cauterization electrode.
FIG. 9 is a view illustrating a tissue dissection device according to a second embodiment.
FIGS. 10 and 11 are views illustrating configurations of the tissue dissecting device in a state where a forceps head is closed or opened.
FIGS. 12 and 13 are views schematically illustrating dissecting states of the anatomical plane using the tissue dissecting device according to the second embodiment.
FIGS. 14 and 15 are views illustrating configurations in which an opening and closing direction of the forceps head is variable.

### DETAILED DESCRIPTION OF THE INVENTION

Hereinafter, an exemplary embodiment of the present invention will be described with reference to the accompanying drawings.

In the detailed description of the embodiments, the elements or configurations obvious to those skilled in the art, such as a known configuration, will be omitted. In addition, it is apparent that the shape, size, number, spacing or the like of an elements in the accompanying drawings may be reduced or exaggerated for convenience of description.

The present invention relates to a dissection device for tissue to apply a dissecting pressure so as to easily and quickly find out an anatomical plane of a human tissue and to allow the tissue to be quickly dissected along the anatomical plane.

FIGS. 2 to 8 illustrate a first embodiment of the present invention, in which a configuration of a tissue dissection device 100 according to the first embodiment is applied to an electrocautery.

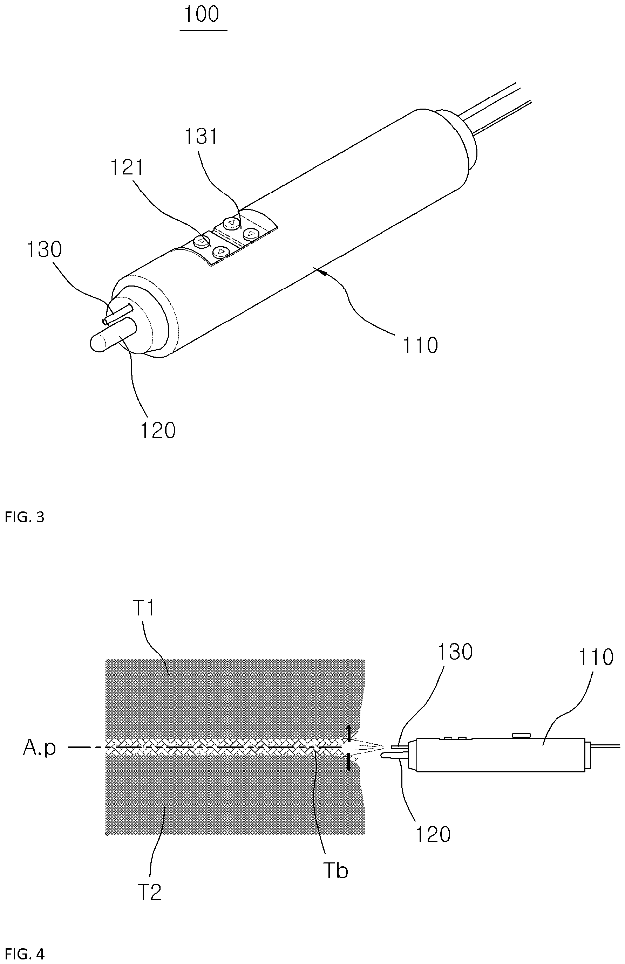

It is well known that the electrocautery demotes an apparatus configured to tear a tissue around a cauterization electrode using thermal energy by locally transmitting a specific current generated by a power supply apparatus to a biological tissue through a cauterization electrode 120 provided in a device body unit 110. As illustrated in FIGS. 2 and 3, the tissue dissection device 100 of the first embodiment includes the device body unit 110 and the cauterization electrode 120 as a configuration of the electrocautery, and further includes a dissecting pressure injection tube 130.

The device body unit 110 may be gripped and manipulated by a doctor, and may have various shapes such as a pen or a gun.

The cauterization electrode 120 is a portion provided at a front end of the device body unit 110 to receive electric power from a power supply (not shown) of the electrocautery, and perform an electro-cauterization by generating heat due to resistance on a part coming into contact with the human tissue. The cauterization electrode 120 may have a blade shape illustrated in FIG. 2 or a probe shape illustrated in FIG. 3.

The cauterization electrode 120 may cut the tissue using strong heat or scorch the tissue using weak heat by adjusting the electric power. Accordingly, the device body unit 110 may be provided with an electric power controller 121 configured to control a power supply of the cauterization electrode 120 according to a required surgical condition such as tissue resection and hemostasis.

The dissecting pressure injection tube 130 is provided in the device body unit 110, and may be provided outside the cauterization electrode 120 to protrude from a front end of the device body unit 110 as shown in FIG. 2. In addition, the dissecting pressure injection tube 130 may be disposed inside the cauterization electrode 120 while passing therethrough other than the outside of the cauterizing electrode 120.

The dissecting pressure injection tube 130 is supplied with a fluid having a predetermined pressure from a fluid supplier (not shown) to inject the fluid toward a front of the cauterization electrode 120, thereby applying a dissecting pressure to the tissue. The fluid supplier may be an air compressor or a CO2 supplier prepared in an operating room.

In addition, a strength of a pressure of the fluid supplied to the dissecting pressure injection tube 130 may be adjusted according to a shape, characteristics or the like of a boundary tissue to be dissected. To this end, the device body unit 110 may be provided with a dissecting pressure manipulation unit 131 configured to adjust the pressure of the fluid. The dissecting pressure manipulation unit 131 may be implemented to adjust the pressure stage by stage, and may be implemented to numerically indicate a pressure value to easily check the pressure.

According to the tissue dissection device 100 having the above-mentioned configuration, when the cauterization electrode 120 is close to a tissue layer as illustrated in FIG. 4, first, the boundary tissue Tb is torn due to the pressure of the fluid injected from the dissecting pressure injection tube 130, and thus a boundary portion between tissue T1 and T2 is opened. Accordingly, when the doctor can easily and accurately find out the boundary portion of the tissue layer to be dissected, that is, a position of the anatomical plane A.p, while checking the torn part.

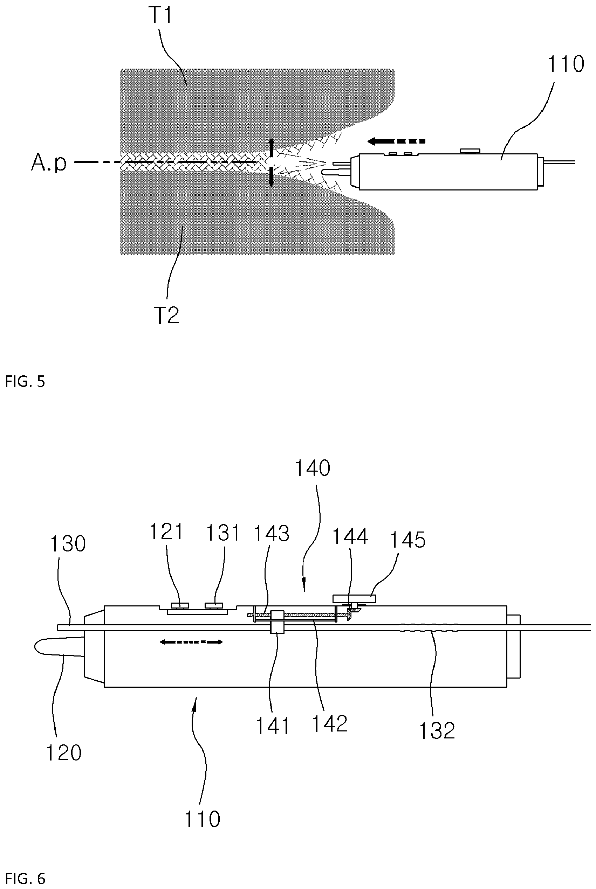

In addition, as illustrated in FIG. 5, when the device body unit 110 enters along the found anatomical plane, the boundary tissue Tb is primarily torn due to the pressure of the fluid injected from the dissecting pressure injection tube 130 and thus the portion between the tissues T1 and T2 is tensioned to be opened. Secondarily, the boundary tissue Tb is completely torn and removed by the cauterization electrode 120. Accordingly, the dissection may be implemented quickly and smoothly.

In addition, when the anatomical plane A.p is opened in advance by the dissecting pressure, a peripheral tissue such as blood vessels may be exposed and recognized beforehand, so that the risk of damage to the peripheral tissue during the dissection may also be minimized.

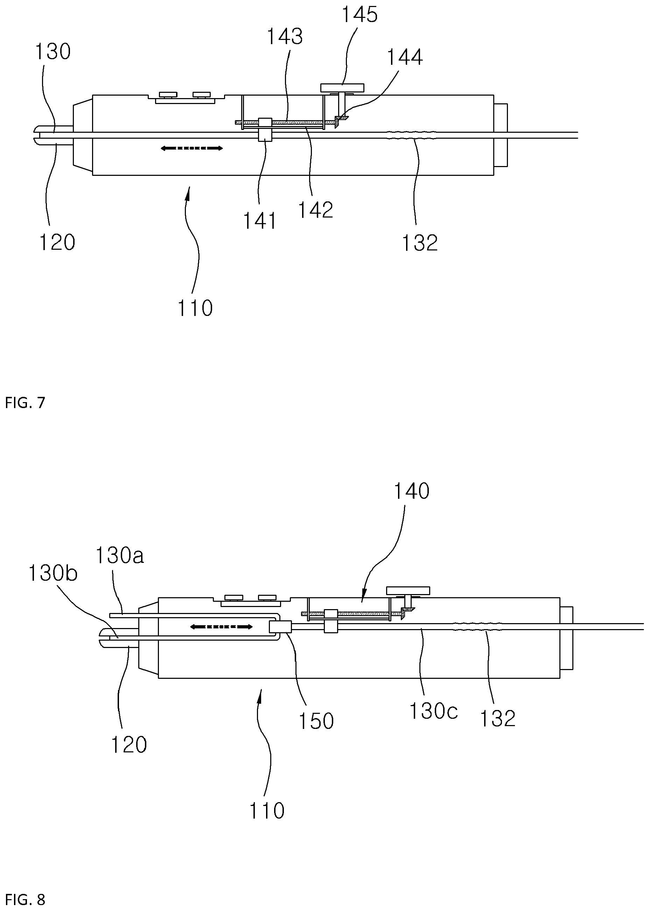

Meanwhile, as illustrated in FIGS. 6 and 7, the tissue dissection device 100 having the above-described configuration further includes an injection tube displacement tool 140 to adjust a relative front-rear position of the dissecting pressure injection tube 130 with respect to the cauterization electrode 120. The dissecting pressure injection tube 130 may further include a flexible portion 132 having a flexible length.

The injection tube displacement tool 140 is configured to provide a forward and backward moving force to the dissecting pressure injection tube 130 from a front end side of the flexible portion 132, and may include, as an example, a moving block 141, a guide 142, a screw member 143, and a rotation manipulating unit 144

The moving block 141 may be fixed to a front end side of the flexible portion 132 of the dissecting pressure injection tube 130. The guide 142 may guide the moving block 141 to move forward and backward while supporting the moving block 141. The screw member 143 may be screwed to the moving block 141 while passing through the moving block 141 and extending in a front-rear direction.

The rotation manipulating unit 144 may apply a rotational force to the screw member 143. The rotation manipulating unit 144 may include a bevel gear for switching a rotation direction, and be combined with a manipulation handle 145 such that the rotational force may be applied to the screw member 143 by rotating the manipulation handle 145.

According to the injection tube displacement tool 140, the moving block 141 may move forward and backward along the guide 142 due to the rotational force of the rotation manipulating unit 144, so that a position of the dissecting pressure injection tube 130 fixed to the moving block 141 may be adjusted in front and rear directions.

According to the above configuration, the position of the dissecting pressure injection tube 130 can be appropriately adjusted in the front and rear directions according to surgical situations. For example, the dissecting pressure injection tube 130 may be retracted to minimize a protruding length so as not to interfere with the electro-cauterization when the non-anatomical plane N.p is resected, and the dissecting pressure injection tube 130 may be advanced to increase the protruding length when the anatomical plane A.p is dissected so that the dissecting pressure can be adjusted to smoothly be applied to the tissue.

In addition, even when the anatomical plane A.p is dissected, the position of the dissecting pressure injection tube 130 may be finely adjusted in the front and rear directions according to a shape, structure or the like of the boundary tissue Tb, so that a pressure injection site at which the dissection is efficiently performed can be appropriately selected.

Meanwhile, as illustrated in FIG. 8, the dissecting pressure injection tube 130 may include an outer injection tube 130a provided outside the cauterization electrode 120, an inner injection tube 130b passing through an inside of the cauterization electrode 120, and a supply injection tube 130c connected to the outer injection tube 130a and the inner injection tube 130b to provide the supplied fluid, and thus may be dually provided outside and inside the cauterization electrode 120.

In addition, a flow path switching unit 150 configured to switch flow paths may be provided at a connection part of the outer injection tube 130a, the inner injection tube 130b, and the supply injection tube 130c to selectively supply the fluid to either of the outer injection tube 130a and the inner injection tube 130b or supply the fluid to both of the outer injection tube 130a and the inner injection tube 130b.

Even when the dissecting pressure injection tube 130 is dually configured as described above, the injection tube displacement tool 140 is configured at the supply injection tube 130c, such that positions of the outer injection tube 130a and the inner injection tube 130b may be adjusted in the front and rear directions.

When the dissecting pressure injection tube 130 is dually configured to switch the flow paths, injection positions of the dissecting pressure may be appropriately selected according to the shape, characteristics or the like of the tissue, so that various human tissues can be efficiently dissected.

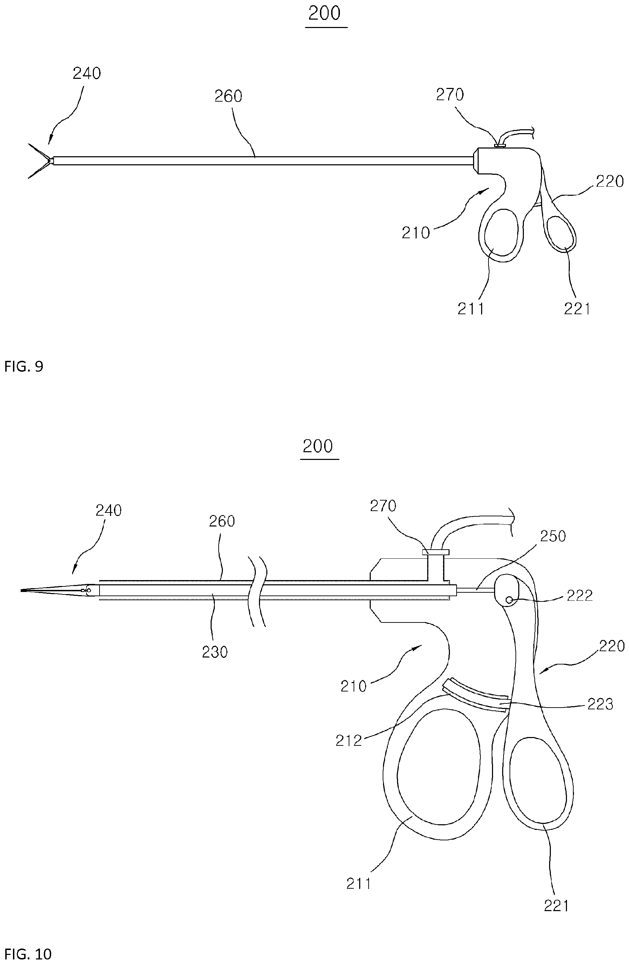

FIGS. 9 to 15 illustrate a second embodiment of the present invention, in which a configuration according to the second embodiment is applied to a dissection device using a trocar.

As illustrated in FIGS. 9 to 11, a tissue dissection device 200 according to the second embodiment includes a device body unit 210, a dissecting operation performing unit, and a dissecting pressure applying unit.

The device body unit 210 is a part manipulated for dissecting a tissue while being gripped by the doctor, in which the device body unit has shape that can be gripped and is formed on a lower side thereof with a grip handle 211 so as to be stably gripped by inserting fingers.

A rotation lever 220 is rotatably coupled to the device body unit 210, in which the rotation lever 220 is rotated so as to be spaced apart from or approach to the grip handle 211, thereby operating the dissecting operation performing unit

A rotating handle 221 may be formed at a lower side of the rotation lever 220 to easily and comfortably perform a rotating manipulation after inserting fingers therein.

In addition, the rotation lever 220 may be provided with a streamlined rotation guide 223 configured to guide the rotation lever 220 to rotate smoothly and stably around a rotation shaft 222. Correspondingly, a streamlined guide rail 212 may be formed inside the device body unit 210 to guide a movement of the rotation guide 223.

The dissecting operation performing unit is a part substantially performing the dissecting operation according to the manipulation of the device body unit 210, and may include a support tube 230, a forceps head 240, and a sliding rod 250.

The support tube 230 is an elongated tube extending from the device body unit 210 by a certain length, and nay enter a human body through a trocar during laparoscopic or robotic surgery.

The forceps head 240 performs a mechanical operation for dissection at a front end of the support tube 230, and may be composed of a pair of forceps pieces 241 and 242 pivotally coupled to the front end of the support tube 230 so as to be opened to each other or closed to each other.

The forceps head 240 is inserted into the tissue in a state where the pair of forceps pieces 241 and 242 are closed, and then opens the tissue while being opened, thereby performing the dissecting operation.

The sliding rod 250 mechanically connects the rotation lever 220 to the forceps head 240, so that the opening and closing operation of the forceps head 240 may be performed according to the manipulation of the rotation lever 220.

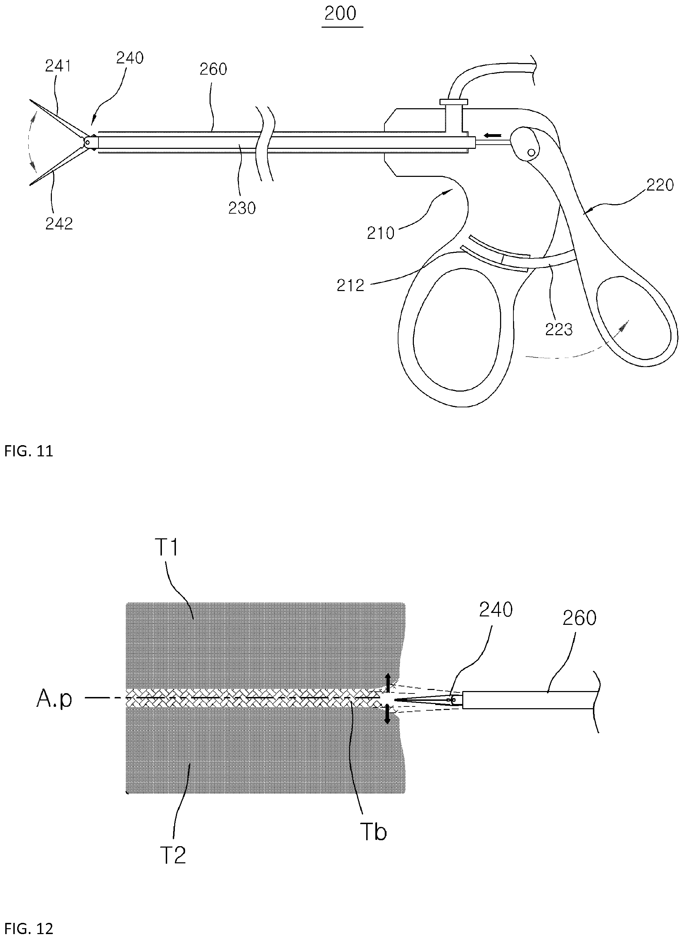

The sliding rod 250 extends along the inside of the support tube 230, in which a front end of the sliding rod 250 may be link-coupled to the forceps head 240, and a rear end may be link-coupled to the rotation lever 120. Accordingly, when the rotation lever 220 is rotatably spaced apart from the grip handle 211 as shown in FIG. 11, the sliding rod 250 moves forward and allows the forceps head 240 to be opened. When the rotation lever 220 is rotated to an initial position as shown in FIG. 10, the sliding rod 250 moves rearward and allows the forceps head 240 to be retracted and closed.

The dissecting pressure applying unit is a part for applying the dissecting pressure to the tissue to open the tissue by the pressure. Unlike the dissecting operation performing unit configured to dissect the tissue by the physical force through the movement of the forceps head 240, the dissection may be performed more easily and smoothly by applying a fluid pressure to the tissue.

The dissecting pressure applying unit includes a dissecting pressure injection tube 260 extending from the device body unit 210 to the position of the forceps head 240 to apply a dissecting pressure by injecting the supplied fluid onto the tissue, and a supply port 270 formed in the device body unit 210 to communicate the dissecting pressure injection tube 260 with the fluid supplier.

For example, the dissecting pressure injection tube 260 may be disposed to extend to surround the support tube 230 along an outer side of the support tube 230. In other words, the dissecting pressure injection tube 260 having an inner diameter relatively larger than an outer diameter of the support tube 230 may be arranged as a double tube structure together with the support tube 230. In addition, the fluid may be supplied through a flow path between an inner peripheral surface of the dissecting pressure injection tube 260 and an outer peripheral surface of the support tube 230.

According to the tissue dissection device 200 having the above-mentioned configuration, the anatomical plane A.p of the tissue may be easily found in the same manner as the first embodiment and the dissection of the tissue using the forceps head 240 may be performed more quickly and smoothly.

In other words, when the forceps head 240 is close to the tissue layer as illustrated in FIG. 12, the boundary tissue Tb is torn and thus the portion between the tissue T1 and T2 is opened due to the fluid pressure injected from the dissecting pressure injection tube 260, so that the position of the anatomical plane A.p may be easily and accurately found.

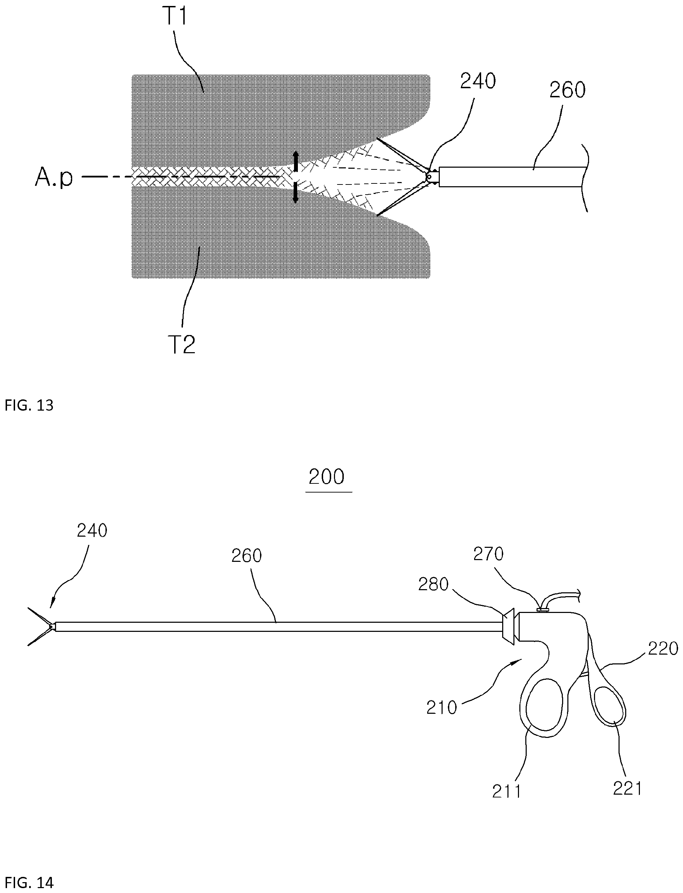

In addition, as illustrated in FIG. 13, when the forceps head 240 is opened by manipulating the rotation lever 220 after the closed forceps head 240 is inserted between the opened tissue T1 and T2, the boundary tissue Tb is primarily torn by the dissecting pressure and thus the portion between the tissues T1 and T2 are further tensioned. Secondarily, the boundary tissue Tb is completely torn and removed by the physical force of the forceps head 240, and thus the tissues T1 and T2 are easily separated from each other, so that the dissection may be implemented more quickly and smoothly.

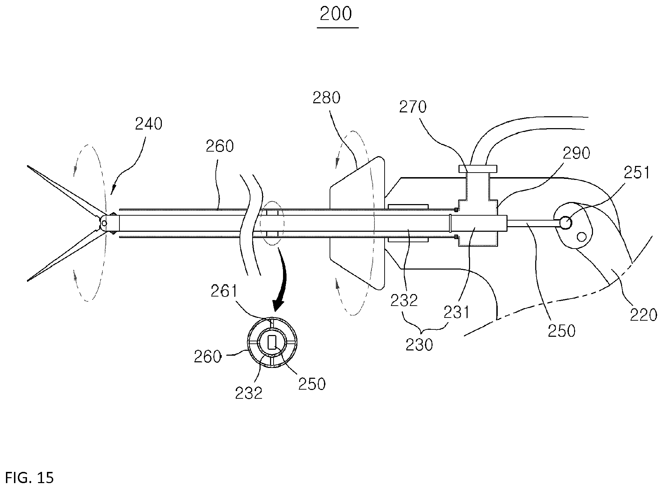

Meanwhile, as illustrated in FIGS. 14 and 15, the tissue dissection device 200 having the above-described configuration may be configured to freely adjust opening and closing directions of the forceps head 240. To this end, the device body unit 210 may further include a rotary handle 280, and the dissecting pressure applying unit may further include a tube connector 290.

The rotary handle 280 is coupled to an outer peripheral surface of the dissecting pressure injection tube 260 and installed to an outer side of a front end of the device body unit 210, so as to provide a rotational force for varying the opening and closing direction of the forceps head 240 while rotating according to the manipulation.

The support tube 230 is composed of a fixed short tube 231 and a rotary long tube 232, in which the fixed short tube 231 is formed at a rear end of the support tube 230 and fixed to the inside of the device body unit 210, and the rotary long tube 232 is formed relatively longer than the fixed short tube 231 and rotatably connected to a front end of the fixed short tube 231 while extending forward from the device body unit 210.

The forceps head 240 is coupled to a front end of the rotary long tube 232. Accordingly, when the rotary long tube 232 rotates, the forceps head 240 also rotates together, so that the opening and closing direction of the forceps head 240 may be variable.

The sliding rod 250 is required to be rotatable in order to rotate the forceps head 240. Accordingly, a rear end of the sliding rod 250 may be provided with a rotation ball member 251 for rotatably supporting the sliding rod 250 onto the rotation lever 220.

The tube connector 290 may be provided between the dissecting pressure injection tube 260 and the supply port 270 so that the fixed supply port 270 and the rotatable dissecting pressure injection tube 260 communicate with each other.

The tube connector 290 may have a sealed space therein and may be fixedly installed inside the device body unit 210, in which the tube connector 290 may be installed to surround the fixed short tube 231 from the outside of the fixed short tube 231.

The supply port 270 may be connected to one side of the fixed tube connector 290, and the dissecting pressure injection tube 260 may be rotatably coupled to the front end of the tube connector 290.

In addition, the dissecting pressure injection tube 260 rotatably connected to the tube connector 290 and the rotary long tube 232 rotatably connected to the fixed short tube 231 may be connected to each other through a connection piece 261, so that the rotary long tube 232 may integrally rotates when the dissecting pressure injection tube 260 rotates. The connection piece 261 may be formed between the dissecting pressure injection tube 260 and the rotary long tube 232 at angular intervals of approximately 90 degrees not to disturb a flow of the fluid through the dissecting pressure injection tube 260.

According to the above configuration, when the rotary handle 280 rotates, the dissecting pressure injection tube 260 rotates together with the rotary long tube 232. Accordingly, the opening and closing direction of the forceps head 240 coupled to the rotary long tube 232 may be variously changed, so that the dissection with respect to the tissue can be performed more conveniently while freely changing the opening and closing direction of the forceps head 240 according to the shape, structure, and characteristics of the tissue to be dissected.

## Claims

1. A dissection device for tissue, the dissection device comprising:
a device body unit to be gripped and manipulated;
a cauterization electrode provided at a front end of the device body unit to perform electro-cauterization by receiving electric power; and
a dissecting pressure injection tube provided in the device body unit to apply a dissecting pressure to a front of the cauterization electrode so as to open the tissue.

2. The dissection device of claim 1, further comprising an injection tube displacement tool configured to adjust a relative front-rear position of the dissecting pressure injection tube with respect to the cauterization electrode by moving the dissecting pressure injection tube forward and backward.

3. The dissection device of claim 2, wherein the dissecting pressure injection tube includes a flexible portion that is stretchable, and the injection tube displacement tool includes:
a moving block fixed to a front end side of the flexible portion of the dissecting pressure injection tube;
a guide configured to guide the moving block to move forward and backward while supporting the moving block;
a screw member screwed to the moving block while passing through the moving block and extending in a front-rear direction; and
a rotation manipulating unit configured to apply a rotational power to the screw member.

4. A dissection device for tissue, the dissection device comprising:
a device body unit to be gripped and manipulated;
a dissecting operation performing unit configured to perform a dissecting operation on the tissue and including a support tube extending forward from the device body unit, and a forceps head provided at a front end of the support tube; and
a dissecting pressure applying unit configured to apply a dissecting pressure to open the tissue and including a dissecting pressure injection tube extending forward from the device body unit.

5. The dissection device of claim 4, wherein the dissecting operation performing unit further includes a sliding rod extending along an inside of the support tube and moving forward and rearward according to a manipulation of the device body unit so as to open and close the forceps head.

6. The dissection device of claim 4, wherein a direction of an opening and closing operation of the forceps head is variable.

7. The dissection device of claim 4, wherein the dissecting pressure injection tube extends along an outer side of the support tube to surround the support tube so that the dissecting pressure injection tube and the support tube have a double tube structure.

8. The dissection device of claim 7, wherein the dissecting pressure injection tube and the support tube are rotatably provided, and the device body unit is provided with a rotary handle configured to rotate the dissecting pressure injection tube and the support tube.

9. The dissection device of claim 8, wherein the support tube includes a fixed short tube fixed to an inside of the device body unit, and a rotary long tube rotatably connected to the fixed short tube and extending forward to be coupled to the forceps head, and the dissecting pressure applying unit further includes a tube connector fixed to an outer side of the fixed short tube in which the dissecting pressure injection tube is rotatably connected to the tube connector.
